# EUROPEAN PATENT SPECIFICATION

(11) **EP 3 013 940 B1**
(45) Date of publication and mention of the grant of the patent: **12.04.2017**
(21) Application number: 14739004.1
(22) Date of filing: 23.06.2014
(51) Int. Cl.: C12N 5/00

(54) **CELL CULTURE ARTICLE AND METHODS THEREOF**
ZELLKULTURARTIKEL UND VERFAHREN DAFÜR
ARTICLE DE CULTURE CELLULAIRE ET PROCÉDÉS ASSOCIÉS

(30) Priority: 24.06.2013 US 201361838452 P
(43) Date of publication of application: 04.05.2016
(60) Divisional application: 17157940.2
(73) Proprietor: Corning Incorporated, Corning, New York 14831 (US)
(72) Inventor: HENRY, David, F-77590 Fontaine le Port (FR); HERVY, Martial, F-77250 Veneux Les Sablons (FR); WALERACK, Corinne, F-77250 Veneux Les Sablons (FR)
(74) Representative: Scheuermann, Erik
(86) International application number: PCT/US2014/043624
(87) International publication number: WO 2014/209865

(56) References cited:
- EP-A1- 1 123 975
- WO-A1-2011/025445
- WO-A2-2012/158235
- US-A1- 2006 084 759
- SRIVASTAVA SWATI ET AL: "Development of a Novel Polygalacturonic Acid-Gelatin Blend Scaffold Fabrication and Biocompatibility Studies for Tissue-Engineering Applications", INTERNATIONAL JOURNAL OF POLYMERIC MATERIALS, GORDON AND BREACH, NEW YORK, NY, US, vol. 61, no. 9, 1 January 2012 (2012-01-01), pages 679-698, XP009180145, ISSN: 0091-4037, DOI: 10.1080/00914037.2011.610070 [retrieved on 2013-03-14]

## Description

### BACKGROUND

The disclosure generally relates to a cell culture article, such as microcarriers, and methods of making and using the article.

### SUMMARY

In embodiments, the present disclosure provides a cell culture article, such as a substrate having a chemically modified surface, and methods of making and using the article.

In embodiments, the disclosure provides a wholly synthetic cell culture article that enables cell culture in chemically defined medium or serum free medium, and allows for the harvest of the cultured cells without using any protease.

In a preferred embodiment, the substrate is a microcarrier. The microcarrier of the disclosure is particularly suitable for large scale cell propagation in chemically defined medium. The microcarrier can support cell growth in a chemically defined medium.

### BRIEF DESCRIPTION OF THE DRAWINGS

In embodiments of the disclosure:
Figs. 1A and 1B, respectively, show exemplary phase contrast microscopy image of the adhesion and growth of human bone marrow-derived mesenchymal stem cells (hMSC) on PGA microcarriers coated with an adhesion polymer, Synthemax II ("PGA-SMII") in stirred culture after 3 days in spinner flasks, and a graph (1B) of cell expansion measured after 5 days in spinner flasks.
Fig. 2 shows a phase contrast microscopy image of cell release from PGA-SMII microcarriers after 5 minutes treatment with 100U pectinase/5 mM EDTA.
Fig. 3 shows a graph of cumulated cell expansion of hMSC grown in spinner flasks on PGA-SMII microcarriers along 6 passages.
Fig. 4 shows a chart of expression of mesenchymal specific markers measured by flow cytometry on hMSC cells grown for 40 days on PGA-SMII microcarriers.
Figs. 5A to 5C show phase contrast microscopy images of the differentiation of different lineages of hMSC cells on PGA-SMII microcarriers.
Fig. 6 shows phase contrast microscopy images of hMSC adhesion in static conditions on esterified pectin (20-32%) coated with SMII or PGA-SMII beads.
Figs. 7A to 7E show a summary of phase contrast microscopy images of hMSC seeded in static conditions for non-optimal processes on various bead surfaces.
Figs. 8A to 8C show phase contrast microscopy images of cell release from PGA-SMII microcarriers with selected treatments.
Fig. 9 shows an exemplary adhesion polymer; a peptide conjugated polymer structure, p(MAA-PEG₄-VN) of the prior art.
Fig. 10 shows another exemplary adhesion polymer; a peptide conjugated polymer structure, p(HEMA-co-MAA-PEG₄-VN) of the prior art.

### Detailed Description

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments of the claimed invention.

In embodiments, the disclosed apparatus and the disclosed method of making and using the apparatus provide one or more advantageous features or aspects, including for example as discussed below. Features or aspects recited in any of the claims are generally applicable to all facets of the invention. Any recited single or multiple feature or aspect in any one claim can be combined or permuted with any other recited feature or aspect in any other claim or claims.

### Definitions

"Wholly synthetic" or "fully synthetic" refers to a cell culture article, such as a microcarrier or surface of a culture vessel, that is composed entirely of synthetic source materials and is devoid of any animal derived or animal sourced materials. The disclosed wholly synthetic cell culture article eliminates the risk of xenogeneic contamination.

"Include," "includes," or like terms means encompassing but not limited to, that is, inclusive and not exclusive.

"About" modifying, for example, the quantity of an ingredient in a composition, concentrations, volumes, process temperature, process time, yields, flow rates, pressures, viscosities, and like values, and ranges thereof, or a dimension of a component, and like values, and ranges thereof, employed in describing the embodiments of the disclosure, refers to variation in the numerical quantity that can occur, for example: through typical measuring and handling procedures used for preparing materials, compositions, composites, concentrates, component parts, articles of manufacture, or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture.

"Optional" or "optionally" means that the subsequently described event or circumstance can or cannot occur, and that the description includes instances where the event or circumstance occurs and instances where it does not.

"Consisting essentially of" or "consisting of" in embodiments can refer to, for example:
a cell culture article, including:
   a substrate comprising a polygalacturonic acid compound selected from at least one of:
      pectic acid;
      partially esterified pectic acid having a degree of esterification from 1 to 40 mol%,
      or salts thereof; and
      an adhesion polymer on the surface of the selected polygalacturonic acid compound, where the adhesion polymer on the surface of the polygalacturonic acid compound is, for example, a polymer having a conjugated polypeptide of the formula poly(HEMA-co-MAA-PEG₄-VN) copolymer, and the polygalacturonic acid compound is optionally crosslinked;
   a method for harvesting cultured cells, including:
      culturing cells; and
      contacting the cultured cells on the surface of the above mentioned culture article with a mixture of pectinase and a chelator, such as EDTA, to separate the cells from the article, as defined herein.

The article, the method of making the article, and the method of using the article, of the disclosure can include the components or steps listed in the claim, plus other components or steps that do not materially affect the basic and novel properties of the compositions, articles, apparatus, or methods of making and use of the disclosure, such as a particular article configuration, particular additives or ingredients, a particular agent, a particular structural material or component, a particular irradiation or temperature condition, or like structure, material, or process variable selected.

The indefinite article "a" or "an" and its corresponding definite article "the" as used herein means at least one, or one or more, unless specified otherwise.

Abbreviations, which are well known to one of ordinary skill in the art, may be used (e.g., "h" or "hrs" for hour or hours, "g" or "gm" for gram(s), "mL" for milliliters, and "rt" for room temperature, "nm" for nanometers, and like abbreviations).

Specific and preferred values disclosed for components, ingredients, additives, dimensions, conditions, and like aspects, and ranges thereof, are for illustration only; they do not exclude other defined values or other values within defined ranges. The apparatus and methods of the disclosure can include any value or any combination of the values, specific values, more specific values, and preferred values described herein, including explicit or implicit intermediate values and ranges.

Trypsin is frequently applied to dissociate adhesive cells from the substratum once cultured cells reach confluence. For example, U.S. Pat. No. 4,994,388, discloses a method for culturing and harvesting anchorage-dependent cells employing microcarrier beads coated with collagen. Once growth is complete, the collagen is digested off of the microcarrier, and the cultured cells are separated from the insoluble microcarrier. However, due to the proteolytic activity of trypsin, cell surface proteins are often cleaved, which may lead to dysregulation of the cell functions. It has been reported that trypsin is able to induce proteome alteration and cell physiological changes. Huang, et al., reported that trypsinization down-regulated growth- and metabolism-related protein expressions and up-regulated apoptosis-related protein expressions, implying that trypsin used for cell subculture had an adverse effect on cell physiology (see Hsiang-Ling Huang et al., "Trypsin-induced proteome alteration during cell subculture in mammalian cells", Journal of Biomedical Science, 2010, 17:36.).

As another example of deleterious effect of proteases, it is also known that treatment of cells with proteases such as trypsin remove antigens from cancer cells and might make them unusable to develop vaccines for anti-cancer therapies. Lokhov, et al., reported that trypsinization releases glycoproteins and sugars from the cell surface (see "A sialomucopeptide liberated by trypsin from the human erythrocyte," Nature, 1960; 188:1011-2, Gasic, G. "Removal and regeneration of the cell coating in tumour cells," Nature, 1962; 196:170, and Uhlenbruck, G., in "Action of proteolytic enzymes on the human erythrocyte surface," Nature, 1961; 190:181 and in "The isolation of living cells from animal tissues," International Review of Cytology, 1956; 7:587-647), thereby leading to a loss of antigenic properties (see David, J.R., et al., "The In Vitro, Desensitization of Sensitive Cells by Trypsin," J Exp Med., 1964;120:1189-200, 40, Weiss, L, et al., "The demonstration of rupture of cell surfaces by an immunological technique," Exp Cell Res., 1963; 30:331-8, Osunkoya, B.O., et al., "Synthesis and fate of immunological surface receptors on cultured Burkitt lymphoma cells," Int J Cancer, 1969; 4(2):159-65, Takeichi, N, et al., "Accelerated regeneration of trypsin-treated surface antigens of simian virus 40-transformed BALB/3T3 cells induced by X-irradiation," Cancer Res., 1976; 36(4):1258-62). In particular, Takeichi, et al., showed that trypsin treatment of SV40-transformed 3T3 cells decreased their antigenicity in footpad assays. Similarly, it has been demonstrated that polyoma virus-transformed cells treated with trypsin failed to induce a delayed hypersensitivity reaction against tumor-specific antigens in footpad swelling assays (see Molinari, J.A., et al., "Modification of surface membrane antigens by trypsin," Proc Soc Exp Biol Med., 1975; 148(4):991-4).

U.S. Pat. No. 5,100,799, Mundt, et al., discloses a method for releasing cell cultures from microcarriers in which a trypsin solution is directed through a container with the microcarriers in a flow-through process. The patent mentions that the released cells must be immediately withdrawn from the carrier, with the trypsin solution being inactivated, removed, or both, after leaving the container to prevent deleterious effect of the protease on the harvested cells. Mundt mentioned that a "high percentage of the cells is released rather quickly and therefore remains in the trypsin solution for a long time. This results in the disadvantage that the action of the trypsin exerts an adverse influence on these cells. Cell growth and cell resettlement capability on new microcarriers that is particularly adversely affected. As a consequence, harvesting cells without trypsin or in the absence of trypsin is highly desirable.

Attempts to replace trypsin by a non-proteolytic enzyme which digests polysaccharide beads was reported in U. S. Patent 6,378,527 (and WO 0056251), to Hungerford, et al., entitled "Cell-culture and polymer constructs". Hungerford mentioned that "the most common method of cell harvesting used is by trypsinization, which may not completely retrieve cells from the microcarriers. Therefore they proposed a microcarrier on which cells are grown and are subsequently separated from the microcarrier by enzymatically digesting the microcarrier. More specifically, chondrocytes were grown on dextran microcarrier beadlets and then the beads were digested using dextranase to separate the chondrocytes from the carrier". This patent also described a dextran microcarrier that can be digested by dextranase but does not mention that the bead can be made of pectinic acid which can be digested by pectinase as in the present disclosure. In addition, Hungerford does not mention how to grow cells in chemically defined medium or serum free medium. Indeed, the microcarrier used was collagen I coated dextran beads, i.e., Cytodex 3 and expansion of the cells on the microcarriers was done in Dulbecco's modified Eagle Medium supplemented with fetal calf serum, and not in chemically defined and serum free medium.

More recently, WO/2011/025445, described dextran beads that are digested by dextranase as previously reported by Hungerford, et al., (*supra.*) WO/2011/025445 also described starch-coated microcarrier that can be digested by amylase. The '445 application mentions that the cell-binding ligands promoting cell attachment must be covalently grafted to the cell culture surface, which surface has been activated with a bifunctional reagent. It is also mentioned that the ligands are attached to the degrading polysaccharide via a specific ligand, such as allylglycidyl ether or an analogous bifunctional reagent. The '445 application described dextran microcarriers and starch-coated microcarrier that can be digested by dextranase or amylase, respectively, but does not mention that the bead can be made of pectinic acid which can be digested by pectinase. Additionally, the '445 application does not teach how to grow cells in chemically defined medium or serum free medium. Still further, the '445 application does not teach that cell attachment can be easily accomplished by coating the carrier with an adhesion polymer such as a synthetic peptide polymer without any chemical derivatization.

Hamilton Global Eukaryotic Microcarrier (GEM™) (see for example, website: hamiltoncompany.com) is another type microcarrier commercially available and composed of an alginic acid core with embedded magnetic particles and a surface which can be covalently bound to substrates for cell attachment and growth. Unfortunately, the microcarrier is coated with a thin molecular layer of gelatin to create a surface suitable for cell culture. According to the manufacturer, removing cells from the GEM is easily done with either Trypsin or Accutase, which will dissolve the microcarrier and gently dissociate cells allowing for the collection of a single cell suspension. As a consequence, although this type of alginic acid based microcarrier could potentially be digested by adding a non-proteolytic enzyme, e.g., alginate lyase, in practice, the cells have to be harvested using a proteolytic enzyme.

JP3771510 mentions an internal regenerative material of tissue characterized as being composed of a cell adhesive active material (A) and a bioabsorptive material (B). (A) is preferably a polypeptide (A1) which is synthesized by gene recombination microorganisms. A list of suitable naturally occurring bioabsorptive material is given which includes pectic acid. This patent application does not mention that a microcarrier can be prepared with ionotropically crosslinked polygalacturonic acid (PGA). In addition the cell adhesive active material is synthesized by gene recombination microorganisms and not prepared by chemical synthesis.

A fully synthetic microcarrier that enables cell expansion in serum free condition, in a chemically defined medium, and allowing cell harvesting without adding protease, as disclosed in the present application would be useful.

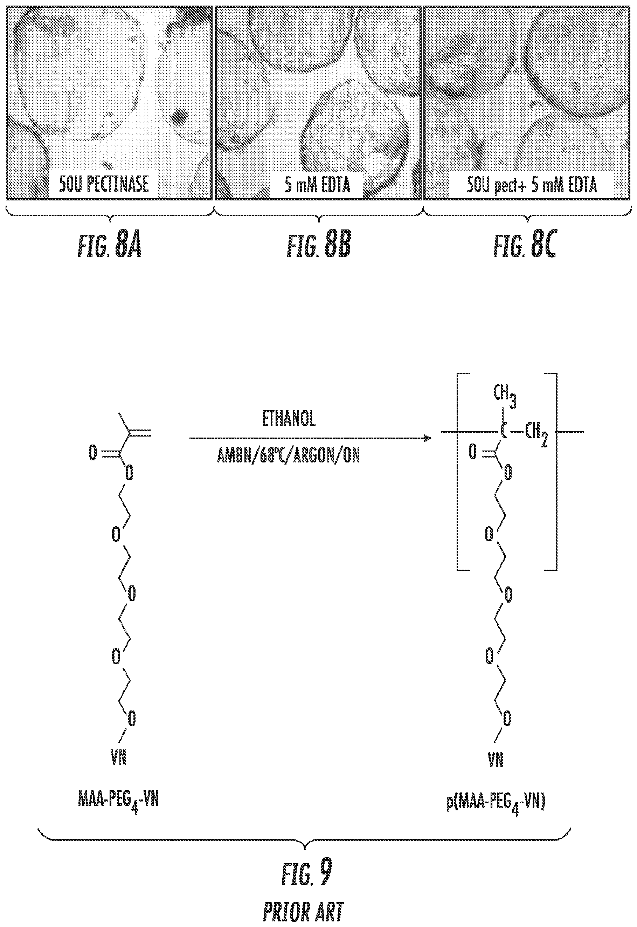

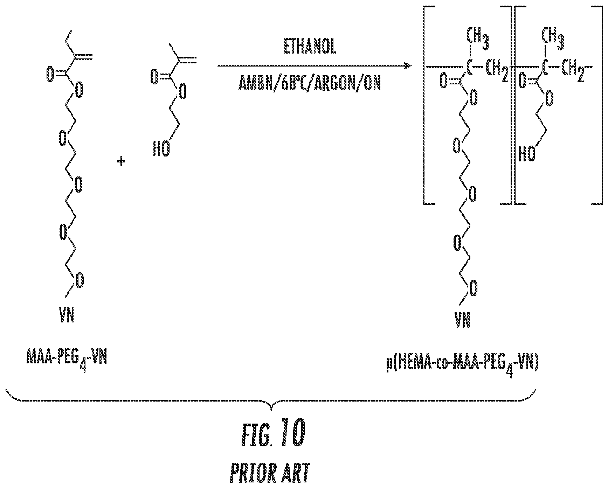

Commonly owned and assigned US patent application number 12/420735 now US Patent Publication No. US20130071916, to Frutos, et al., published Mar. 21, 2013 (also published as WO2012158235) mentions a method for coating a surface of a cell culture article includes dissolving a polymer having a covalently attached polypeptide in an aqueous solution to produce a polymer solution. The polymer is formed from mono-functional monomers (i.e., no monomers having di- or higher- functionality). The weight percentage of the polypeptide relative to the polymer conjugated to the polypeptide is sufficiently high to render the polymer conjugated to the polypeptide water soluble. The aqueous solution is substantially free of organic solvents. The method further includes (i) disposing the polymer solution on the surface of the cell culture article to produce a coated article; and (ii) subjecting the coated article to sufficient heat or electromagnetic radiation to attach the polymer conjugated to a polypeptide to the surface of the cell culture article. Exemplary peptide conjugated polymers include, for example, p(MAA-PEG₄-VN), and p(HEMA-co-MAA-PEG₄-VN) (shown in the present application as Fig. 9 and Fig. 10).

In embodiments, the synthetic microcarriers of the disclosure can be prepared, for example, from at least an ionotropically cross-linked biopolymer selected, for example, from:
pectic acid, also known as polygalacturonic acid (PGA); or
partially esterified pectic acid (also known as pectinic acid) (PE PGE); or salts thereof, or a mixture thereof.

When partially esterified pectic acid is selected, the degree of esterification can be, for example, about 40 mol% or less, such from 1 mol% to 39 mol%, 5 to 35 mol%, 10 to 30 mol%, including intermediate values and ranges.

In embodiments, the microcarrier of the disclosure enables rapid and complete cell harvesting by contacting the microcarrier, on which the cells were grown, with pectinase, and optionally a chelating agent, e.g., EDTA, and without the need of adding any protease.

In embodiments, the microcarrier of the disclosure is advantageously and preferably made of pectic acid or pectinic acid beads, or salts thereof, having microsphere dimensions, which beads are functionalized with cell adhesion promoting peptides, and more preferably functionalized by coating with a synthetic polymer bearing adhesion peptides. The adhesion peptides enable bio-specific adhesion of the cultured cells. The disclosed microcarrier is suitable for large-scale expansion and recovery of adherent cells in serum free culture.

In embodiments, the articles, materials, and methods of the disclosure are advantaged by, for example:
the wholly synthetic cell culture substrate prevents the risk of xenogeneic contamination;
the cells can be readily harvested from the PGA bead without a protease by, for example, using a pectinase and a chelator such as EDTA, which pectinase dissolves the PGA bead and gently disassociates the cells, and results in a single cell suspension for further downstream processing;
the synthetic cell culture substrate, as e.g., a microcarrier, maximizes surface area-to-volume ratio, which enables large-scale cell culture in a compact footprint; and
the synthetic cell culture substrate can be prepared by, for example, in a straightforward coating process that reduces the consumption of reagents and reduces labor.

The cell culture substrate composition can comprise, for example, at least pectic acid, partially esterifed pectic acid, or salts thereof, and mixtures thereof; and at least one peptide promoting the attachment of anchorage dependent cells.

Preferably the composition comprises a peptide-polymer conjugate, which conjugate promotes the attachment of anchorage dependent cells, more preferably the peptide-polymer conjugate is poly (meth)acrylate or poly(meth)acrylamide copolymer comprising an adhesion peptide, and more preferably the peptide-polymer conjugate is Synthemax^{®}II.

In embodiments, the composition is preferably ionically crosslinked. The crosslinking is preferably obtained by an ionotropic gelation method, and more preferably the crosslinking is obtained by at least one internal gelation method.

In embodiments, the disclosure provides a cell culture article, comprising:
a substrate comprising a polygalacturonic acid compound selected from at least one of:
   pectic acid;
   partially esterified pectic acid having a degree of esterification from 1 to 40 mol%,
   or salts thereof; and
   an adhesion polymer on the surface of the polygalacturonic acid compound.

In embodiments, the polygalacturonic acid compound can be, for example, covalently cross linked, ionically cross linked, or mixtures thereof.

In embodiments, the partially esterified pectic acid can be, for example, an alkyl carboxy ester having an alkyl group having from 1 to 10 carbon atoms.

In embodiments, the adhesion polymer on the surface of the polygalacturonic acid compound can be, for example, a polypeptide.

In embodiments, the adhesion polymer on the surface of the polygalacturonic acid compound can be, for example, a polymer having a conjugated polypeptide.

In embodiments, the adhesion polymer on the surface of the polygalacturonic acid compound is a polymer having a conjugated polypeptide selected from at least one of:
poly(MAA-PEG₄-VN) homopolymer, that is, Synthemax^{®} I (SMI);
poly(HEMA-co-MAA-PEG₄-VN) copolymer, that is, Synthemax^{®} II (SMII);
or mixtures thereof,
where:
MAA is methacrylic acid;
HEMA is hydroxyethylmethacrylate;
PEG₄ is a polyethylene glycol tetra oligomer; and
VN is a conjugated vitronectin polypeptide.

Synthemax^{®} I polymer and Synthemax^{®} II copolymer and methods of making are disclosed in the abovementioned USSN 13/420,735.

In embodiments, the adhesion polymer can be, for example, present in an amount of from 0.1 to 30 weight % based on the total weight of the article.

In embodiments, the adhesion polymer can, for example, promote the attachment of anchorage dependent live cells to the substrate.

In embodiments, the substrate can be, for example, a microcarrier particle.

In embodiments, the disclosure provides a method for harvesting cultured cells,
comprising:
culturing cells; and
contacting the cultured cells on the surface of the above mentioned culture article with a mixture of pectinase and a chelator to separate the cells from the article.

In embodiments, the method can further comprise, for example, isolating the separated cells from the composition.

In embodiments, the chelator can be, for example, EDTA, like multi dentate chelators, or mixtures thereof.

In embodiments, the contacting can be accomplished, for example, free of a protease.

In embodiments, the method of making the abovementioned cell culture composition
or article, comprising:
coating the surface of the substrate comprised of any polygalacturonic acid compound disclosed herein with an adhesion polymer.

In embodiments, the method can further comprise, for example, crosslinking the selected polygalacturonic acid compound by, for example, ionic crosslinking, by internal gelation, or a combination thereof.

In embodiments, the substrate can be, for example, a microcarrier.

### PGA Polymers

The synthetic microcarrier of the disclosure can be made of at least one ionotropically cross-linked polysaccharide selected from, for example, pectic acid, also known as polygalacturonic acid (PGA), or a salt thereof, or partly esterified pectic acid (PE PGA) known as pectinic acid, or a salt thereof. When pectinic acid is selected, the degree of esterification is preferably less than about 40 mol% since a higher degree of esterification makes bead formation by ionotropic crosslinking ineffective. Without being bound by theory it is believed that a minimum amount of free carboxylic acid groups may be called for to obtain an acceptable level of ionotropic crosslinking.

The beads used as the microcarrier of the disclosure are preferably prepared from a mixture of pectic acid or pectinic acid. Pectic acid can be formed by the hydrolysis of certain esters of pectins. Pectins are cell wall polysaccharides which have a structural role in plants. They are predominantly linear polymers based on a 1,4-linked alpha-D-galacturonate backbone, interrupted randomly by 1,2-linked L-rhamnose. The average molecular weight is from about 50,000 to about 200,000 Daltons.

Two major sources of pectins are, for example, from citrus peel (mostly lemon and lime) or apple peels, and can be obtained by extraction thereof.

The polygalacturonic acid chain of pectins can be partly esterified with methyl groups and the free acid groups may be partly or fully neutralized with monovalent ions such as sodium, potassium, or ammonium ions. Polygalacturonic acids partly esterified with methanol is called pectinic acids, and salts thereof are called pectinates.

The degree of methylation (DM) for commercial high methoxyl (HM) pectins typically can be, for example, from 60 to 75 mol% and those for low methoxyl (LM) pectins can be from 1 to 40 mol%, 10 to 40 mol%, and 20 to 40 mol%, including intermediate values and ranges.

The microcarriers of the disclosure are preferably prepared from the LM pectins and preferably the polygalacturonic acid contains less than 20 mol% methoxyl groups, and more preferably the polygalacturonic acid has no or only negligible methyl ester content as pectic acids. For simplicity both pectinic acid having no or only negligible methyl ester and low methoxyl (LM) pectins are referred to as PGA in the disclosure.

### Cross-linking by Ionotropic gelation of PGA

The polygalacturonic acid beads of the disclosure can be cross-linked to prevent their dissolution into the cell culture medium. Crosslinking is preferably performed by ionotropic gelation as described below, and more preferably by internal gelation. Ionotropic gelation is based on the ability of polyelectrolytes to cross link in the presence of multivalent counter ions to form crosslinked hydrogels.

The gelation of these polyelectrolytes, e.g., alginate and pectate, results from the strong interactions between divalent cations, such as calcium, and blocks of either galacturonic or guluronic acid residues for PGA and alginate, respectively (see "Polysaccharide Gel Layers in the Presence of Ca2+ and K+ Ions: Measurements and Mechanisms", Alexis J. de Kerchove, et al., Biomacromolecules, 2007, 8, 113-121). The ionotropic gelation process is simple and inexpensive.

In embodiments, some chemical crosslinking can be performed but the level of chemical crosslinking, being irreversible, should be sufficiently low, for example, less than about 10 to 20 mol%, so as to maintain the digestibility of the bead by the pectinase. From prior studies it is known that the structure of the gel can significantly influence degradation where, for example, a more highly crosslinked gel can lead to overall longer degradation times. Crosslinking reduces pore size of the hydrogel and restricts enzyme access, and consequently reduces the digestion efficiency.

It has been reported that ionotropically crosslinked pectate can provide many advantages over calcium alginate gels. Calcium pectate gels were a better alternative to calcium alginate gel in multiple applications of immobilized cells (see P. Gemeiner, et al., Progress in Biotechnology, Vol 11, 1996, Pages 76-83) when such gels were used to encapsulate cells. The highest mechanical strength, lowest shrinkage, and best stability towards monovalent cations, of PGA gel beads can be accounted for because the PGA contains 100 mol% of galacturonic residues being able to interact with the divalent cations to form crosslinking sites, which is not the case for alginate.

As a consequence, calcium pectate gels are less sensitive to ions and chemical agents, and especially chelating anions which destroy calcium alginate gels.

The stability of the preferred ionotropically crosslinked PGA beads (compared to alginate beads) is a significant advantage especially when the beads are used as substrate for cell culture. Such high stability prevents disaggregation of the beads during the cell culture, especially in microcarrier stirred-suspension culture.

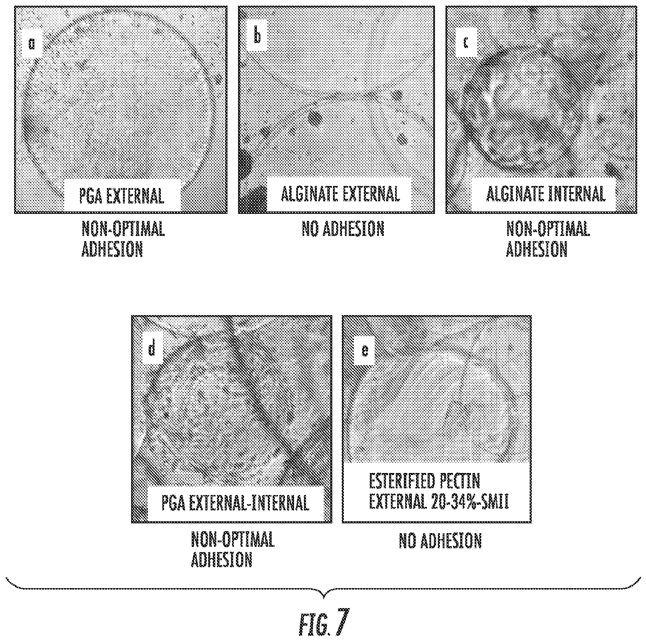

Comparative Example 2 and Figs. 7B and 7C show that cells do not grow on either alginate beads prepared by external gelation (see Fig. 7 B) or internal gelation (see Fig. 7 C) that are subsequently coated with Corning^{®} Synthemax^{®}II.

### Methods for ionotropically crosslinking beads

PGA bead preparation can be accomplished by, for example, forming the microcarrier bead from droplets of PGA solution that fall into a gelling bath containing a multivalent cation, e.g., calcium, magnesium, zinc, and like cations, to permit the ionotropic gelation to occur as described above.

### External and internal ionotropic gelation

There are at least two different approaches or procedures for making ionotropically cross-linked PGA beads: external and internal gelation. It is also known that internal and external gelation can be combined together. In external gelation, a PGA aqueous solution is dispensed drop-wise through a needle into a solution of divalent cations, such as calcium or magnesium, which induces ionic cross-linking of the PGA polymer. It has been reported that externally-gelled beads are usually inhomogeneous with a higher concentration of polymer near the bead surface, thus reducing the porosity of the resulting bead. Comparative Example 8 shows that such beads made by external crosslinking of PGA polymer with calcium and subsequently coated with Corning Synthemax^{®} II do not support cell growth in serum free medium (see Fig.7A). In addition, industrial scale-up is complicated, resulting in an awkward production system. Furthermore, it can be difficult to obtain smaller size microparticles when using such an external gelation process.

In the internal gelation, which has been reported to be scalable, beads are formed via gelation of a PGA aqueous solution containing an insoluble calcium salt dispersed in the aqueous phase and emulsified within oil. Gelation is initiated by addition of an oil-soluble acid to reduce the pH of the PGA solution and releasing soluble Ca²⁺ or Mg²⁺ from the insoluble salt.

Various calcium salts can be used, for example, oxalate, tartrate, phosphate, carbonate, citrate, and like organic and inorganic anions, or combinations thereof. Ionic crosslinking can be easily achieved by using a salt of a metal, for example: magnesium, calcium, zinc, strontium, barium, and like cations, or combinations thereof. A preferred divalent cation is calcium. Calcium is an insolubilizing cation widely used as a crosslinking agent for polysaccharide hydrogels. Ionically crosslinked polysaccharide hydrogels have been used, for example, for wound dressings. Calcium salts have been widely used as ionic crosslinking agent for therapeutic purposes.

Non-limiting examples of techniques to prepare PGA beads, include for example: dripping or extrusion with a syringe, jet breakup or pulverization, for which bead formation is achieved by a coaxial air stream that pull droplets from a needle tip into a gelling bath; an electrostatic bead generator, which uses an electrostatic field to pull droplets from a needle tip into a gelling bath; a magnetically driven vibrator causing the break-up; and a jet cutting technique for which bead formation is achieved by means of a rotating cutting tool, which cuts a jet into uniform cylindrical segments (technology available from Genialab, GmbH). These segments form spherical beads while falling down into a gelling bath. Spinning Disk Atomization Bead formation can be achieved with a spinning disk atomizer. Emulsification can be also employed as described below.

### Emulsification / Internal Gelation Method for PGA beads preparation (a preferred method)

The most common method of preparation of microparticles of PGA or alginate comprises emulsification of solution of the PGA or alginate and subsequent gelation of the droplets. The emulsification/internal gelation technique to form alginate microspheres has been described previously (see Poncelet, D., et al., Production of alginate beads by emulsification/internal gelation. I. Methodology. Appl. Microbiol. Biotechnol, 38, 39-45 1992b).

Small diameter PGA beads are formed via internal gelation of an aqueous PGA solution or dispersion emulsified within an organic phase, e.g., synthetic or vegetable oils, and containing dispersed calcium salt. Gelation is initiated by addition of an oil-soluble acid to reduce the pH of the PGA solution and releasing soluble Ca²⁺ from the insoluble salt. A preferred salt is calcium carbonate. The preferred calcium salt can be precipitated calcium carbonate having a narrow particle size distribution and small size particles, which provide a more stable dispersion of the calcium carbonate particles in the dispersed aqueous phase. A median particle size can be, for example, from about 0.01 to 0.5 microns, preferably from 0.04 to 0.15 microns, including intermediate values and ranges. A typical average particle size can be, for example, from about 0.04 to about 0.08 microns, including intermediate values and ranges. A suitable oil can be, for example, vegetable oil, paraffin oil, fatty alcohol, and like oils, or combinations thereof. A particularly suitable oil is n-octanol.

Surfactant is usually added to stabilize emulsion droplets against coalescence. Non-limiting oil soluble surfactants having a low HLB, can include, for example, Span 85 sorbitan triester, Span 60 sorbitan monostearate-SM, Span 80 sorbitan oleate, Tergitol NP-4, Tergitol NP-7, Tergitol 15-S3, Triton X-15, Triton X-35, Triton X-45, polymeric dispersing agents such as cellulose-acetate-butyrate, or like surfactants, and mixtures thereof.

### Post treatment of PGA beads promoting cell attachment

PGA beads, due to their hydrogel nature and negative charge, do not readily support cell attachment without specific treatment. The PGA beads can be functionalized with moieties promoting cell adhesion, for example, with peptides. Peptides containing amino acid sequences potentially recognized by proteins from the integrin family, or leading to an interaction with cellular molecules able to sustain cell adhesion, are candidates for functionalizing the present microcarriers. Preferred peptides can be, for example, selected from BSP, vitronectin, fibronectin, laminin, collagen, and like peptides, and mixtures thereof. Particularly preferred peptides are vitronectin (VN) and BSP peptides having the following sequences: Ac-Lys-Gly-Pro-Gln-Val-Thr-Arg-Gly-Asp-Val-Phe-Thr-Met-Pro-NH₂ (seq. ID No.: 1), and Ac-Lys-Gly-Gly-Asn-Gly-Glu-Pro-Arg-Gly-Asp-Thr-Tyr-Arg-Ala-Tyr-NH₂ (seq. ID No.: 2), respectively.

The microcarrier of the disclosure can be, if desired, advantageously functionalized by simple physical adsorption of polymers such as adhesive peptides.

Suitable polymers promoting cell adhesion preferably comprise a synthetic polymer. Examples of such a synthetic polymer bearing peptide that promotes cell adhesion and growth includes Corning Incorporated's Synthemax^{®}II. Eliminating chemical derivatization from the manufacturing process by using physical adsorption of an adhesion promoting polymer appears attractive since chemical derivatization is time consuming, labor intensive, requires a large amount of reagents, and generates a large amount of waste chemicals.

The coating prepared from polymers comprising adhesive peptides is particularly effective when performed on beads that have been cross-linked by internal gelation, and, in contrast, fails on beads cross-linked by external gelation.

As demonstrated in Comparative Example 8, for beads prepared by external gelation, the coating of peptide copolymer fails to support cell expansion on PGA beads prepared by external gelation.

Without being bound by theory it is believed that the surface compactness of externally formed gels offers a higher resistance to diffusion of the peptide polymer used for coating in contrast to the better absorption/adsorption of the peptide polymer on porous and more homogeneous gel formed by internal gelation. It is believed that a more stable adsorption of the peptide polymer is achieved and results in a more efficient cell attachment and better cell growth.

### Non-proteolytic enzyme suitable for harvesting the cell

For a bead made of pectic polymer, a non-proteolytic enzyme suitable for harvesting the cell, digesting the microcarrier, or both, can include pectinolytic enzymes or pectinases, which are a heterogeneous group of related enzymes that hydrolyze the pectic substances, present mostly in plants.

Pectinases (polygalacturonase) are enzymes that break down complex pectin molecules to shorter molecules of galacturonic acid. Pectinase catalyzes the liberation of pectic oligosaccharides (POS) from polygalacturonic acid.

Pectinases are produced by fungi, yeast, bacteria, protozoa, insects, nematodes and plants.

Commercially available sources of pectinases are generally multi-enzymatic, such as Novozyme Pectinex^{™} ULTRA SPL, a pectolytic enzyme preparation produced from a selected strain of *Aspergillus aculeatus*. It contains mainly polygalacturonase, (EC 3.2.1.15) pectintranseliminase EC 4.2.2.2) and pectinesterase (EC: 3.1.1.11). Pectinases are known to hydrolyze pectin. They may attack methyl-esterified pectin or de-esterified pectin. The EC designation and number is the Enzyme Commission classification scheme for enzymes based on the chemical reactions the enzymes catalyze.

### Partial or total bead digestion

Depending of the digestion time, temperature, and amount of pectinolytic enzyme added, the extent of digestion beads can be selected or predetermined. We have observed that cells detach from the surface before than the whole bead was fully digested. Therefore, it is possible to harvest the cells without complete digestion of the beads or after complete bead digestion. In the former, the beads must be separated from the cells by means of a physical process, e.g., filtration, decantation, centrifugation, and like processing, or combinations thereof.

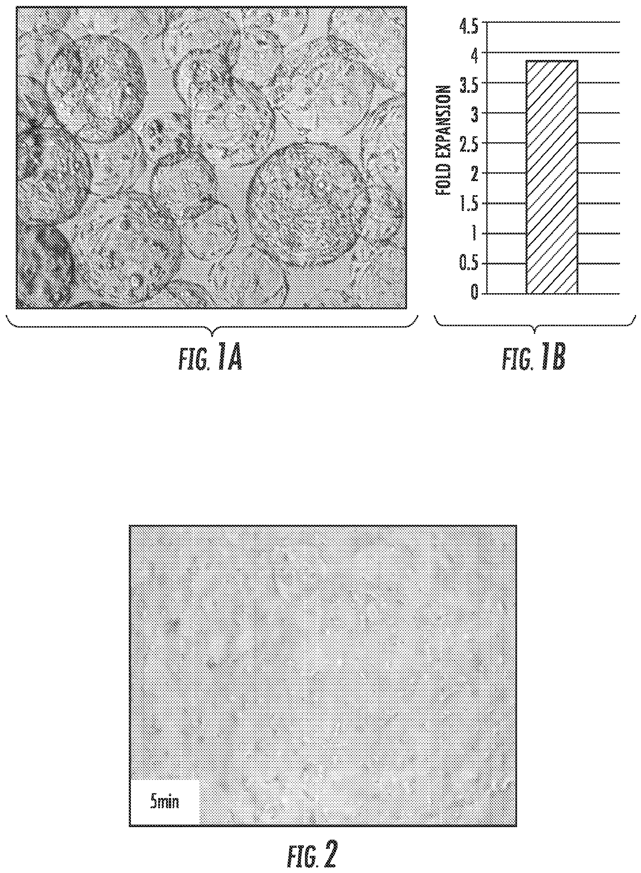

Referring to the Figures, Figs. 1A and 1B, respectively, show a phase contrast microscopy image (1A) of the adhesion and growth of hMSC on PGA microcarriers coated with Synthemax II (PGA-SMII) in stirred culture after 3 days in spinner flasks, and the graph (1B) corresponds to the cell expansion measured after 5 days in spinner flasks.

Fig. 2 shows a phase contrast microscopy image of cell release from PGA-SMII microcarriers after 5 minutes treatment with 100U pectinase/5 mM EDTA.

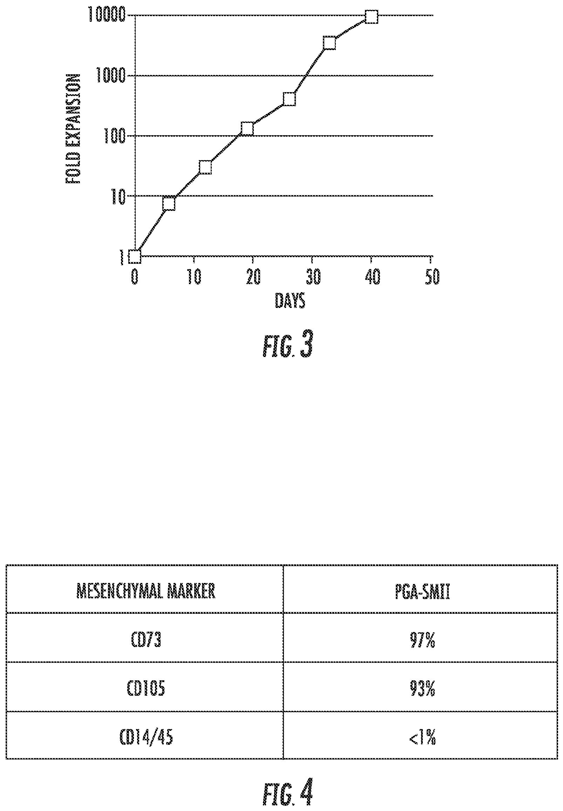

Fig. 3 shows a graph of cumulated cell expansion of hMSC grown in spinner flasks on PGA-SMII microcarriers along 6 passages.

Fig. 4 shows a chart of expression of mesenchymal specific markers measured by flow cytometry on hMSC cells grown for 40 days on PGA-SMII microcarriers.

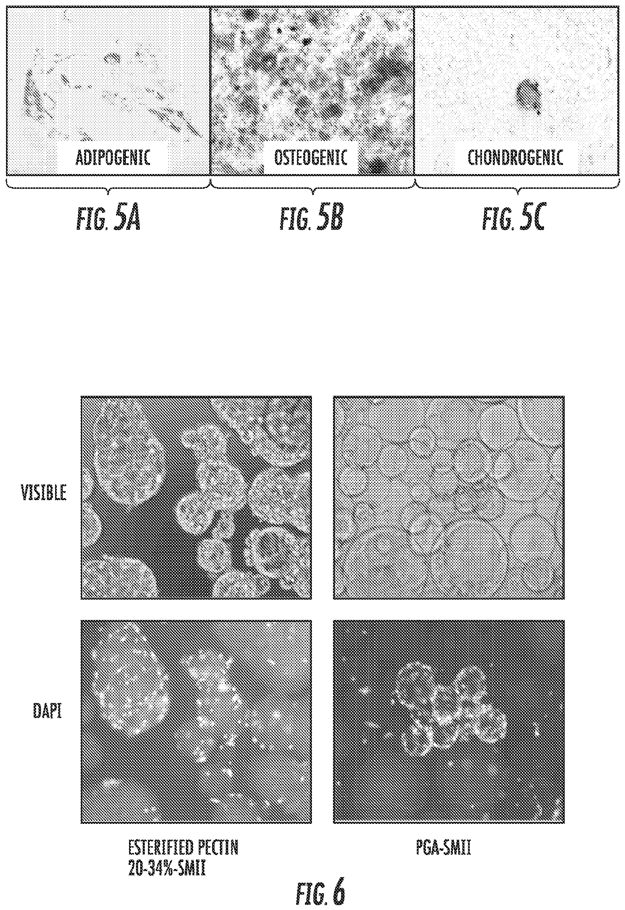

Figs. 5A to 5C show phase contrast microscopy images of the differentiation of hMSC grown for 40 days on PGA-SMII microcarriers to adipogenic (5A), osteogenic (5B), and chondrogenic (5C) lineages and stained respectively with red, oil O, Alizarin red, and alcian blue.

Fig. 6 shows phase contrast microscopy images of hMSC adhesion in static conditions on esterified pectin (20-32%) coated with SMII or PGA-SMII beads. Images in the visible channel or after DAPI staining are presented.

Figs. 7A to 7E show a summary of phase contrast microscopy pictures of hMSC seeded in static conditions for non-optimal processes on various bead surfaces: PGA beads prepared by external gelation using CaCl₂ solution as the hardening solution (7A); alginate beads prepared by external gelation using CaCl₂ solution as the hardening solution (7B); alginate beads prepared by emulsification /internal gelation method (7C); PGA beads prepared by external gelation followed by internal gelation method (7D); and esterified pectin (20-32%) prepared by external gelation(7E). All beads were coated with SMII.

Figs. 8A to 8C show phase contrast microscopy images of cell release from PGA-SMII microcarriers treated for 5 minutes with 50U pectinase, 5 mM EDTA or a 50U pectinase/5mM EDTA mix. Cell release as single cells is observed only for pectinase and EDTA/mixed treatment (8C) illustrating the cooperative or synergistic effect of the reagents in combination.

Figs. 9 and 10 respectively show exemplary adhesion polymers having peptide conjugated polymer structures, p(MAA-PEG₄-VN) and p(HEMA-co-MAA-PEG₄-VN) of the commonly owned prior art.

### EXAMPLES

The following examples serve to more fully describe the manner of using the above-described disclosure, and to further set forth best modes contemplated for carrying out various aspects of the disclosure. These examples do not limit the scope of this disclosure, but rather are presented for illustrative purposes. The working example(s) further describe(s) aspects of how to prepare and use the disclosed cell culture articles.

### Example 1

**PGA Microsphere prepared by emulsification/internal gelation technique** Polygalacturonic acid (PGA) sodium salt, Sigma catalog no. P3850, was dispersed into DI water at 80 °C for 16 hrs under stirring to give a 2.0% (w/v) PGA suspension. A suspension of microcrystalline CaCO₃, Sigma cat no. 21061, was prepared by suspending CaCO₃ powder in DI water (5%, w/v). The pH was adjusted to 6.5 to 7.0 by adding glacial acetic acid (one droplet). 500 microliters of this CaCO₃ suspension was added into 20 mL 2.0% (w/v) PGA sodium salt and the mixture was homogenized with an IKA Ultra-Turrax® disperser for a few seconds. This mixture was emulsified into 100 g n-octanol, containing 2% (w/w) of Span®85 by stirring at 260 rpm using a Heidolph RZR 2020 mixer equipped with an anchor stirrer. The ratio between aqueous PGA and oily phases was about 14/86 (v/v). After 10 min emulsification, 20 mL of n-octanol containing 80 microliters of glacial acetic acid were added and stirring continued for 45 min to permit the solubilization of the calcium carbonate. PGA gelled beads were washed three times with a 4% (w/w) CaCl₂ solution in ethanol. The beads were then washed three times with proof ethanol followed by washing three times with DI water. Beads are stored in DI water prior to coating.

### Example 2

**Coating of PGA Microspheres with** adhesion polymer **Synthemax^{®}II synthetic copolymer** About 9 mL of swollen beads, prepared according to Example 1, were placed in a 50 mL plastic centrifuge tube. A solution of Corning® Synthemax^{®} II - SC copolymer in DI water, 0.25 mg/mL, was also prepared. 33 mL of this Corning^{®} Synthemax^{®} II - SC copolymer solution was added to the swollen PGA beads. The tube was gently shaken and left undisturbed for 30 min at 40°C allowing the synthetic copolymer to functionalize the beads. After cooling the functionalized beads were washed three times with DI water. Beads in DI water were stored at 4°C in sterile containers.

### Comparative Example 3

**Alginate microspheres prepared by emulsification/internal gelation** technique Alginate beads prepared by emulsification/internal gelation were prepared as described in Example 1 except that low viscosity alginic acid sodium salt from brown algae (Sigma cat. no. A2158), was used instead of PGA sodium salt.

### Comparative Example 4

**PGA microspheres prepared by external gelation technique** Polygalacturonic acid (PGA) sodium salt (Sigma cat. no. P3850), was dispersed into DI water at 80 °C for 16 hrs under stirring to give a 2.0% (w/v) PGA suspension. 250 milliliters of a 3 % w/v of calcium chloride water/ethanol, 75/25 v/v, solution, used as coagulation fluid, was placed in a beaker and stirred slowly using a magnetic stirrer. Ten milliliters of the PGA dispersion was fed dropwise, 250mL/h, into the coagulation fluid using a syringe pump equipped with a 30 gauge needle. Beads were hardened in calcium chloride for 60 minutes before being washed twice with Milli-Q water, then were stored in sterile water in sterile containers.

### Comparative Example 5

**Alginate Microspheres prepared by external gelation technique** Alginate microspheres were prepared by external gelation technique as described in Comp Example 4 except that alginic acid sodium salt (Sigma catalog no. A2158), was used instead of PGA sodium salt.

### Example 6

**Expansion of hMSC stirred conditions using the disclosed microcarrier** hMSC were cultured on the PGA microcarriers coated with an adhesion polymer Synthemax II-SC (PGA-SMII) of Example 2 in stirred culture in spinner flasks. Cell morphology and expansion were measured after 5 days are shown on Fig 1. Expected cell morphology and cell expansion was evident. Cells were harvested from the PGA-SMII microcarriers by treatment with 100U pectinase/ 5 mM EDTA for 5 minutes (see Fig. 2). Digestion of the beads and hMSC cell release was evident. Cumulated cell expansion on PGA-SMII microcarriers along 6 passages is shown in the graph of Fig. 3. A 10,000 fold expansion is achieved after about 40 days.

Expression of mesenchymal specific markers was measured by flow cytometry, the expression level of the mesenchymal specific markers CD73 and CD105 was measured over 90%, comparable to cells grown in flasks (not shown). As expected, no expression of negative markers CD14 and CD45 was detected (Fig. 4).

Cells were harvested from microcarriers after 5 passages in xeno-free medium and re-plated on 6-well plates for directed differentiation into adipogenic, osteogenic, and chondrogenic lineages.

The phase contrast microscopy images of Fig. 5 clearly showed that differentiation of the hMSC into each of the three lineages was successful.

### Example 7

Adhesion of hMSC was evaluated in static conditions on beads prepared by emulsification/internal gelation of esterified pectin (20-34%) or PGA and coated with adhesion polymer SMII. Fig 6 microscopic images show that the cells are able to adhere on both beads.

### Comparative Example 8

Adhesion of hMSC seeded in static conditions was evaluated on PGA beads prepared by external gelation as described in Comparative Example 4, alginate beads prepared by external gelation as described in in Comparative Example 5, alginate beads prepared by emulsification/internal gelation method prepared as described in Comparative Example 3 and bead prepared by external gelation of esterified pectin (20-34%). All beads types were coated with SMII to promote cell attachment. Fig. 7 microscopic images show that not all of the bead types support optimal hMSC cell attachment. The data provides evidence that alginate is unsuitable to prepare the microcarrier of the disclosure, and that external gelation is an unsuitable method even when PGA is used.

### Example 9

**Efficiency of EDTA, pectinase, or a mix of both, on cell release** The efficiency of EDTA alone, pectinase alone, or a mixture of both, to release the cells from the beads was tested on adhesion polymer Synthemax^{®}II coated PGA beads. Cells where grown on microcarriers in static conditions for 3 days in mesencult XF medium, and treated with 50 units of pectinase, 5 mM EDTA, or a 50U pectinase 5 mM EDTA mix. As illustrated in Fig. 8, when the cells are treated with only pectinase (8A) or EDTA (8B), cell detachment from the beads was observed but the cells tend to remain associated in aggregates. When pectinase and EDTA (8C) are used as a mixture the cells are detached from the beads and cell aggregates are dissociated and resulted in cell detachment as single cells. For many cell types, it is significant to avoid aggregation when sub-culturing the cells to obtain homogeneous cell seeding and prevent the formation of over-confluent zones in further culture steps. Collecting cells as single cells is also important if cell characterization using methods such as flow cytometry is used.

### Materials and methods for cell culture

### Cells

Human bone marrow-derived mesenchymal stem cells (hMSC) were obtained from STEMCELL Technologies (Cat. No. MSC-001F). For routine maintenance in Mesencult®-XF medium (Cat. No. 05429), cells were grown on Mesencult®-XF attachment substrate coated plates (Cat. No. 05424) from STEMCELL Technologies.

### Pectinase EDTA treatment

Pectinase (Novozyme pectinex Ultra SPL (Sigma P2611)) was diluted in PBS (100U/mL) and complemented with 10 mM EDTA pH8. This release buffer was preincubated at 37°C. For bead digestion and cell release, beads with adhered cells were collected, pelleted by 2 min centrifugation at 1500 rpm, washed in PBS and incubated in pectinase and EDTA solution at 37°C for 5 minutes. If the incubation times are longer then proportionately longer pectinase EDTA treatment durations are indicated.

### Static culture

Microcarriers were resuspended in 1 mL culture medium and transferred to ULA treated 24 wells ULA plates (Corning ref#3473). 50,000 hMSC cells were then seeded directly in the wells. Plates were incubated at 37°C in a cell culture incubator under a 5% CO₂ atmosphere. Culture medium (50-60%) was replaced every other day.

### Spinner flasks cultures

Microcarriers were resuspended in culture medium and transferred to Corning^{®} 125 mL disposable spinner flasks (Corning, Cat. No. 3152). hMSC were seeded directly onto microcarriers in a 15 mL final volume (1E6 cells per spinner flask). Spinner flasks were placed in a cell culture incubator at 37°C under a 5% CO₂ atmosphere, without agitation for 18 to 20 hours. After that, medium volume was adjusted to 35 mL (up to 45 mL performed similarly), and cultures were stirred at 30 rpm every 2 hours for 15 minutes. Culture medium (50 to 60%) was replaced every other day.

Serial passage in Corning® Stemgro® hMSC Medium hMSC seeding was performed as described above. After 7 days, cells were passaged by removing 80% of the culture after gentle homogenization. Fresh PGA - SMII microcarriers were added back to the spinner flask. The final culture volume was adjusted to 35 mL with Corning® Stemgro® hMSC medium, and agitation was started at 30 rpm for 15 minutes every 2 hours. The collected cells-microcarriers were pelleted by centrifugation, washed with dPBS, and treated with pectinase/EDTA. Detached cells were counted using the trypan blue exclusion method with a hemacytometer. Duplicate cell samples were collected for flow cytometry analysis and directed differentiation experiments.

### Flow cytometry

Harvested cells were fixed in 2% paraformaldehyde in dPBS for 10 minutes at room temperature and then washed and stored in dPBS. Cells were incubated in blocking buffer for 15 min at 4 °C, followed by 30 minute incubation at RT with primary antibodies against CD14, CD45, CD73, CD105 (Millipore Chemicon) or corresponding isotype controls (Invitrogen/Molecular Probes) in blocking buffer. After a brief wash, the cells were incubated with 1:200 dilution of corresponding secondary antibodies for 30 minutes at RT in the dark. For each sample, 30,000 events were acquired using BD FACS Calibur Flow Cytometer (BD Biosciences). Histogram overlay subtraction analysis using the BD Cell Quest® Pro software (BD Biosciences) was performed to calculate the percent of marker positive cells.

### Directed differentiation

Cells were harvested from microcarriers after 5 passages in xeno-free medium and re-plated on 6-well plates for directed differentiation into adipogenic, osteogenic, and chondrogenic lineages using the following kits: Adipogenic differentiation kit (Lonza, Cat. PT3102A/B), StemPro® Osteogenic differentiation kit (Gibco, Cat. No. A10072-01) and StemPro® Chondrogenic differentiation kit (Gibco, Cat. No. A10071-01). Stains used: Oil Red Staining Kit (Millipore), Alizarin Red Stain (Millipore), and 1% Alcain Blue Stain (Fisher Scientific). The protocols for differentiation and staining were performed according to manufacturer's recommendations.

The disclosure has been described with reference to various specific embodiments and techniques. However, it should be understood that many variations and modifications are possible while remaining within the scope of the disclosure.

## Claims

1. A cell culture article, comprising:
a substrate comprising a polygalacturonic acid compound selected from at least one of:
pectic acid;
partially esterified pectic acid having a degree of esterification from 1 to 40 mol%, or salts thereof;
and
an adhesion polymer on the surface of the polygalacturonic acid compound.

2. The article of claim 1 wherein the polygalacturonic acid compound is covalently cross linked, ionically cross linked, or mixtures thereof.

3. The article of any of claims 1 to 2 wherein the partially esterified pectic acid comprises an alkyl carboxy ester having an alkyl group having from 1 to 10 carbon atoms.

4. The article of any of claims 1 to 3 wherein the adhesion polymer on the surface of the polygalacturonic acid compound comprises a polypeptide.

5. The article of any of claims 1 to 4 wherein the adhesion polymer on the surface of the polygalacturonic acid compound comprises a polymer having a conjugated polypeptide.

6. The article of any of claims 1 to 5 wherein the adhesion polymer on the surface of the polygalacturonic acid compound is a polymer having a conjugated polypeptide selected from at least one of:
poly(MAA-PEG₄-VN) homopolymer;
poly(HEMA-co-MAA-PEG₄-VN) copolymer;
or mixtures thereof,
where:
MAA is methacrylic acid;
HEMA is hydroxyethylmethacrylate;
PEG₄ is a polyethylene glycol tetra oligomer; and
VN is a conjugated vitronectin polypeptide.

7. The article of any of claims 1 to 6 wherein the adhesion polymer is present in an amount of from 0.1 to 30 weight % based on the total weight of the article, or based on the total weight of the polygalacturonic acid compound or compounds selected.

8. The article of any of claims 1 to 7 wherein the adhesion polymer promotes the attachment of anchorage dependent live cells to the substrate.

9. The article of any of claims 1 to 8 wherein the substrate comprises a microcarrier particle.

10. A method for harvesting cultured cells, comprising:
culturing cells on the surface of the article of claim 1; and
contacting the cultured cells with a mixture of pectinase and a chelator to separate the cells from the article.

11. The method of claim 10, further comprising isolating the separated cells from the article.

12. The method of any of claims 10 to 11 wherein the chelator is EDTA.

13. The method of any of claims 10 to 12 wherein the contacting is accomplished free of a protease.

14. A method of making the cell culture particle of claim 1 comprising:
coating the surface of the substrate comprised of polygalacturonic acid compound with an adhesion polymer.

15. The method of claim 14 further comprising crosslinking the polygalacturonic acid compound.

16. The method of any of claims 14 to 15 wherein crosslinking the polygalacturonic acid compound is accomplished ionically, by internal gelation, or a combination thereof.

17. The method of any of claims 14 to 16 wherein the substrate is a microcarrier.

## Patentansprüche

1. Zellkulturgegenstand, der Folgendes umfasst:
ein Substrat, das eine Polygalakturonsäureverbindung umfasst, die aus mindestens einer der folgenden ausgewählt ist:
Pektinsäure;
teilveresterte Pektinsäure mit einem Veresterungsgrad von 1 bis 40 Mol-% oder Salze davon;
und
ein Adhäsionspolymer auf der Oberfläche der Polygalakturonsäureverbindung.

2. Gegenstand nach Anspruch 1, wobei die Polygalakturonsäureverbindung kovalent verknüpft, ionisch verknüpft oder Mischungen davon ist.

3. Gegenstand nach einem der Ansprüche 1 bis 2, wobei die teilveresterte Pektinsäure einen Alkylcarboxyester mit einer Alkylgruppe mit 1 bis 10 Kohlenstoffatomen umfasst.

4. Gegenstand nach einem der Ansprüche 1 bis 3, wobei das Adhäsionspolymer auf der Oberfläche der Polygalakturonsäureverbindung ein Polypeptid umfasst.

5. Gegenstand nach einem der Ansprüche 1 bis 4, wobei das Adhäsionspolymer auf der Oberfläche der Polygalakturonsäureverbindung ein Polymer mit einem konjugierten Polypeptid umfasst.

6. Gegenstand nach einem der Ansprüche 1 bis 5, wobei das Adhäsionspolymer auf der Oberfläche der Polygalakturonsäureverbindung ein Polymer mit einem konjugierten Polypeptid ist, das aus mindestens einem der folgenden ausgewählt ist:
Poly-(MAA-PEG₄-VN)-Homopolymer;
Poly-(HEMA-co-MAA-PEG₄-VN)-Copolymer
oder Mischungen davon,
wobei:
MAA Methacrylsäure ist;
HEMA Hydroxyethylmethacrylat ist;
PEG₄ ein Polyethylenglykol-Tetraoligomer ist und
VN ein konjugiertes Vitronektin-Polypeptid ist.

7. Gegenstand nach einem der Ansprüche 1 bis 6, wobei das Adhäsionspolymer in einer Menge von 0,1 bis 30 Gew.-%, bezogen auf das Gesamtgewicht des Gegenstands oder bezogen auf das Gesamtgewicht der ausgewählten Polygalakturonsäureverbindung oder -verbindungen, vorliegt.

8. Gegenstand nach einem der Ansprüche 1 bis 7, wobei das Adhäsionspolymer die Anlagerung von verankerungsabhängigen lebenden Zellen an dem Substrat fördert.

9. Gegenstand nach einem der Ansprüche 1 bis 8, wobei das Substrat ein Mikroträgerteilchen umfasst.

10. Verfahren zum Ernten von kultivierten Zellen, das Folgendes umfasst:
Kultivieren von Zellen auf der Oberfläche des Gegenstands nach Anspruch 1 und
Inkontaktbringen der kultivierten Zellen mit einer Mischung von Pektinase und einem Chelatbildner, um die Zellen von dem Gegenstand zu trennen.

11. Verfahren nach Anspruch 10, das weiterhin das Isolieren der getrennten Zellen von dem Gegenstand umfasst.

12. Verfahren nach einem der Ansprüche 10 bis 11, wobei der Chelatbildner EDTA ist.

13. Verfahren nach einem der Ansprüche 10 bis 12, wobei das Inkontaktbringen frei von einer Protease ausgeführt wird.

14. Verfahren zum Herstellen des Zellkulturgegenstands nach Anspruch 1, das Folgendes umfasst:
Beschichten der Oberfläche des Substrats, das aus einer Polygalakturonsäureverbindung besteht, mit einem Adhäsionspolymer.

15. Verfahren nach Anspruch 14, das weiterhin das Vernetzen der Polygalakturonsäureverbindung umfasst.

16. Verfahren nach einem der Ansprüche 14 bis 15, wobei das Vernetzen der Polygalakturonsäureverbindung ionisch, durch interne Gelierung oder eine Kombination davon ausgeführt wird.

17. Verfahren nach einem der Ansprüche 14 bis 16, wobei das Substrat ein Mikroträger ist.

## Revendications

1. Article de culture cellulaire, comprenant :
un substrat comprenant un composé d'acide polygalacturonique sélectionné parmi au moins l'un des éléments suivants :
acide pectique ;
acide pectique partiellement estérifié ayant un degré d'estérification de 1 à 40 % en mole, ou des sels de celui-ci ; et
un polymère d'adhérence à la surface du composé d'acide polygalacturonique.

2. Article selon la revendication 1, où le composé d'acide polygalacturonique est réticulé de manière covalente, réticulé de manière ionique ou des mélanges de ces procédés.

3. Article selon l'une quelconque des revendications 1 ou 2, où l'acide pectique partiellement estérifié comprend un alkyl carboxy ester possédant un groupe alkyle possédant de 1 à 10 atomes de carbone.

4. Article selon l'une quelconque des revendications 1 à 3, où le polymère d'adhérence à la surface du composé d'acide polygalacturonique comprend un polypeptide.

5. Article selon l'une quelconque des revendications 1 à 4, où le polymère d'adhérence à la surface du composé d'acide polygalacturonique comprend un polymère possédant un polypeptide conjugué.

6. Article selon l'une quelconque des revendications 1 à 5, où le polymère d'adhérence à la surface du composé d'acide polygalacturonique comprend un polymère possédant un polypeptide conjugué sélectionné parmi au moins l'un des éléments suivants :
poly(MAA-PEG₄-VN) homopolymère ;
poly(HEMA-co-MAA-PEG₄-VN) copolymère ;
ou des mélanges de ceux-ci,
où :
MAA est l'acide méthacrylique ;
HEMA est l'hydroxyéthylméthacrylate ;
PEG₄ est un polyéthylène glycol tétra oligomère ; et
VN est un polypeptide de vitronectine conjugué.

7. Article selon l'une quelconque des revendications 1 à 6, où le polymère d'adhérence est présent dans une quantité allant de 0,1 à 30 % en poids sur la base du poids total de l'article, ou bien sur la base du poids total du composé d'acide polygalacturonique ou des composés sélectionnés.

8. Article selon l'une quelconque des revendications 1 à 7, où le polymère d'adhérence favorise la fixation, au substrat, de cellules vivantes dépendantes d'un support.

9. Article selon l'une quelconque des revendications 1 à 8, où le substrat comprend une particule microporteuse.

10. Procédé de récolte des cellules de culture, comprenant :
la culture de cellules à la surface de l'article selon la revendication 1 ; et
la mise en contact des cellules de culture avec un mélange de pectinase et d'un chélateur pour séparer les cellules de l'article.

11. Procédé selon la revendication 10, comprenant en outre l'isolation des cellules séparées par rapport à l'article.

12. Procédé de l'une quelconque des revendications 10 ou 11, où le chélateur est l'EDTA.

13. Procédé selon l'une quelconque des revendications 10 à 12, où la mise en contact se déroule sans protéase.

14. Procédé de fabrication de l'article de culture cellulaire selon la revendication 1 comprenant :
le revêtement de la surface du substrat constitué du composé d'acide polygalacturonique avec un polymère d'adhérence.

15. Procédé selon la revendication 14, comprenant en outre la réticulation du composé d'acide polygalacturonique.

16. Procédé selon l'une quelconque des revendications 14 ou 15, où la réticulation du composé d'acide polygalacturonique se déroule de manière ionique, par gélation interne, ou une combinaison de ces procédés.

17. Procédé selon l'une quelconque des revendications 14 à 16, où le substrat est un microporteur.
